Europäisches Patentamt

European Patent Office

Office européen des brevets

(11) Publication number: 0 285 052
A2

# EUROPEAN PATENT APPLICATION

(21) Application number: 88104941.5

(22) Date of filing: 26.03.88

(51) Int. Cl.⁴: **C07K 15/00** , C12P 21/00 ,
//C12N5/00,C12N15/00,
A61K39/395,G01N33/569,
G01N33/577,(C12P21/00,
C12R1:91)

(30) Priority: 28.03.87 JP 74989/87

(43) Date of publication of application:
05.10.88 Bulletin 88/40

(84) Designated Contracting States:
BE DE ES FR GB NL SE

(71) Applicant: **THE GREEN CROSS CORPORATION**
**15-1, Imabashi-1-chome Higashi-ku**
**Osaka-shi**
**Osaka 541(JP)**

(72) Inventor: **Kanoh, Yoshiaki**
**1-1-508, Furuichi 3-chome Johtoh-ku**
**Osaka-shi Osaka(JP)**
Inventor: **Taniguchi, Tomokuni**
**The Green Cross Corporation Ryokufu-ryo**
**24-9**
**Kitanakaburi 1-chome Hirakata-shi**
**Osaka(JP)**
Inventor: **Hayashi, Masako**
**13-25-322, Karita 2-chome Sumiyoshi-ku**
**Osaka-shi Osaka(JP)**
Inventor: **Uemura, Yahiro**
**18-215, Mitsuya-cho 5-chome**
**Hirakata-shi Osaka(JP)**

(74) Representative: **von Kreisler, Alek,**
**Dipl.-Chem. et al**
**Deichmannhaus am Hauptbahnhof**
**D-5000 Köln 1(DE)**

(54) Monoclonal antibody.

(57) A monoclonal antibody having the following characteristics which are produced by hybridomas prepared using a bile duct cancer-derived established cancer cell line (HEK) as the immunogen.

1) Ig class (subclass): IgM($x$)
2) Type of recognizing antigen: Secretory antigen
3) Reactivity with cancer cells: Positive for at least esophageal cancer cell line TE-1, esophageal cancer cell line TE-2, esophageal cancer cell line TE-7, esophageal cancer cell line TE-9, esophageal cancer cell line TE-10, lung cell line PC-3, gastric cancer cell line MKN-45, gastric cancer cell line KATO-III, bile duct cancer cell line HEK, endometrial cancer cell line ISHIKAWA and colorectal cancer cell line COLO-201.

The monoclonal antibodies of this invention have a potential for clinical application to diagnostic drugs useful

for early detection of cancer or prognostic trace study and to targeting therapy in which the monoclonal antibody is conjugated with radio isotopes or anti-cancer agents.

## MONOCLONAL ANTIBODY

BACKGROUND OF THE INVENTION

1. Field of the Invention

The present invention relates to monoclonal antibodies having particular characteristics which are produced by hybridomas prepared using a bile duct cancer-derived cell line (HEK) as the immunogen.

2. Description of Prior Art

The ultimate purpose of cancer research is to search for substances exhibiting anticancer action and establish a method of early detection of cancer. A wide variety of drugs to cancer have conventionally been developed. However, all of these affect not only cancer cells but also normal tissues and normal cells; at present, even if the drugs are very effective, their use is restricted due to their severe adverse reactions.

Immune reactions (antigen-antibody reactions) have extremely high specificity; however, when the conventional polyclonal antibodies are used, it is difficult to recognize those immune reactions which are discriminated by very minor antigenic determinants such as interlymphocytic subsets. The monoclonal antibody technology developed by Milstein et al. [Köhler, G. and Milstein, C.: Nature, 256, 495 (1975)] has overcome this difficulty; it is expected that the specific elimination of cancer cells without causing damage on normal cells can be achieved by obtaining monolclonal antibodies which recognize cancer-specific or cancer-associated antigens on cancer cells. In addition, it seems that diagnostic drugs or assay reagents utilizing such monoclonal antibodies can detect cancer-associated or cancer-specific antigens with high sensitivity since they show no cross reaction with normal serum components.

DESCRIPTION OF THE INVENTION

The purpose of this invention is to provide monoclonal antibydies which specifically react with particular cancer antigens.

This invention comprises monoclonal antibodies which specifically react with esophageal cancer, lung cancer, gastric cancer, colorectal cancer, bile duct cancer and endometrial cancer.

Accordingly, the monoclonal antibodies of this invention are produced by hybridomas prepared using a bile duct cancer-derived established cell line (HEK) as the immunogen and have the following characteristics.

1) Ig class (subclass): IgM($x$)

2) Type of recognizing antigen: Secretory antigen

3) Reactivity with cancer cells: Positive for at least esophageal cancer cell line TE-1, esophageal cancer cell line TE-2, esophageal cancer cell line TE-7, esophageal cancer cell line TE-9, esophageal cancer cell line TE-10, lung cancer cell line PC-3, gastric cancer cell line MKN-45, gastric cancer cell line KATO-III, bile duct cancer cell line HEK, endometrial cancer cell line ISHIKAWA and colorectal cancer cell line COLO-201.

The monoclonal antibodies of this invention are classified into three varieties: the monoclonal antibody which is positive for esophageal cancer cell line TE-3, esophageal cancer cell line TE-6, gastric cancer cell line MKN-28 and colorectal cancer cell line COLO-320DM, as well as reactive with the cancer cell lines shown in 3) above; the monoclonal antibody which is also positive for esophageal cancer cell line TE-5; and the monoclonal antibody which is also positive for esophageal cancer cell line TE-3, lung cancer cell line PC-9, renal cancer cell line NRC-12 and ovarian teratoma cell line PA-1.

The monoclonal antibodies of this invention are produced by what is called cell fusion technique, i.e., it is produced by forming a hybridoma between an antibody-producing cell and a myeloma cell, cloning the hybridoma thus obtained, and selecting a clone which produces an antibody exhibiting specificity to the above-mentioned cancer cell lines (namely, specific antigens having the above-mentioned characteristics). The procedure thereof may be in accordance with the conventionally known method except that the following are used as cells for immunization.

The antibody-producing cells are splenic cells, lymph node cells or B-lymphocytes derived from an animal immunized with an immunogen, for example, an antigen obtained from an established cancer cell line. As the established cancer cell line, bile duct cancer-derived established cancer cell line HEK is used.

This cell line is characterized in that it produces tumor markers such as carcinoembryonic antigen (hereinafter referred to as CEA), the antigenic substance which reacts with the monoclonal antibody disclosed in the specification of EPC Patent No. 171083 (hereinafter referred to as KM01) (hereinafter referred to as KM01 antigen), and the antigenic substance disclosed in the specification of EPC Patent No. 165811 (also called sialated Lewis X; hereinafter referred to as CSLEX1 antigen).

Examples of the immunogens include an already established cancer cell line, dispersed cancer cells from surgical specimen or extract thereof and a cell line newly established from surgical specimen of cancer patient.

As the bile duct cancer-derived line HEK, the line established from a bile duct cancer-derived tissue is preferably used.

Animals to be immunized are exemplified by mice, rats, horses, goats and rabbits.

The immunogens of this invention are used for immunizing animals after being mixed with, for example, Freund's complete adjuvant. Immunization is done by subcutaneously, intramuscularly or intraperitoneally injecting the immunogen to the animal in an amount corresponding to about $10^5$ to $10^8$ cells per injection; 2 to 5 times of immunization are done at intervals of about 1 to 4 weeks starting from the first immunization and the final immunization is conducted about 1 to 4 months later. About 3 to 5 days after the final immunization, antibody-producing cells are separated from the immunized animal.

Examples of usable myeloma cell lines include those derived from mice, rats, humans and other animals. Examples of such myeloma cell lines include mouse myeloma lines P3U1 and X-63-Ag8-6.5.3. It is preferable that the antibody-producing cells and myeloma cells are derived from the same animal species.

Cell fusion is for example conducted by the method described in G. Galfre: Nature, 266, 550 (1977) or a similar method, in which about 1 to 3 minutes of reaction is carried out at a temperature of 30 to 40°C in the presence of 30 to 50% polyethylene glycol (average molecular weight: 1,000 to 4,000).

The cells obtained by the cell fusion are subjected to screening of clones which produce the desired monoclonal antibody. That is, fused cells are for example cultured on a microplate and determined the antibody titer of the culture supernatant of each well in which proliferation occurred by, for example, the enzyme-linked immuno sorbent assay (hereinafter referred to as ELISA) and the wells in which the desired antibody has been produced are selected. These wells are subjected to further cloning by, for example, limiting dilution method to thereby obtain the desired clone. This clone is intraperitoneally transplanted to BALB/C mice previously primed with pristane and grown therein; 10 to 14 days later, ascites fluid containing a high concentration of the monoclonal antibody is collected and assayed. The hybridoma can also grow in a serum-free medium containing bovine serum albumin (0.1 to 1%); for example, the clone is grown in a serum-free medium (e.g. RITC55-9 medium) containing 0.5% bovine serum albumin and the culture supernatant is collected.

The recovery of the monoclonal antibody produced by the selected clone can easily be achieved by applying a conventionally known methods of immunoglobulin purification, for example, ammonium sulfate fractionation, polyethylene glycol fractionation, ethanol fractionation and/or anion exchangers.

It is inferred that the monoclonal antibodies obtained according to this invention recognize secretory antigens and cancer-specific antigens. They do not react with normal cell-derived cultured cells, red blood cells, white blood cells or normal tissues. Accordingly, the monoclonal antibodies of this invention have a potential for clinical application to in vivo and in vitro diagnostic drugs useful for early detection of cancer or prognostic trace study and to targeting therapy in which the monoclonal antibody is conju gated with radio isotopes or anti-cancer agents.


Example 1

(1) Preparation of immunogen:

(1-i) Establishment of bile duct cancer cell line

From surgical specimens provided by Kobe University (see Table 1), 4 to 5 blocks of about $5 \times 10 \times 2$ mm in size were cut. After washing with a phosphate buffer, the blocks were suspended in 30 ml of a phosphate buffer containing 0.05% collagenase (Wako Pure Chemical Co.) and 100 U/ml Trasylol (Bayer Co.), and shaked for 1 hour at 37°C. After centrifugation (1000 rpm $\times$ 5 min.), the supernatant was discarded. The residual precipitate was washed once, after which it was suspended in 30 ml of MEM medium containing 10% fetal bovine serum containing 1000 U/ml Dispase (Sanko Pure Chemical Co.), and shaked for 1 hour at 37°C. The suspension was kept standing for 3 minutes to separate the supernatant and precipitate from each other and after washing, each of which was suspended in RPMI-1640 medium containing 20% fetal bovine serum. The suspension was transferred into a cultivation flask and incubated at 37°C in 5% $CO_2$. The medium was exchanged at intervals of 7 to 10 days, and after an epithelioid cell colony was noted, the medium was exchanged at a frequency of 2 times a week. After the 8th generation, subculture was continued with the fetal bovine serum content being reduced to 10%. Subsequent subculture was done in RPMI-1640 medium containing 10% fetal bovine serum. The culture supernatant of this cell line (named HEK) was found to contain 256 to 512 ng/ml of CEA as determined by the hemagglutination inhibition method using CEA-sensitized blood cells (hereinafter referred to as HAI method) and 1:256 KM01 antigen as determined with KM01-RPHA reagent. A value of 1:32 was exhibited when the monoclonal antibody disclosed in the specification of EPC Patent No. 165811 (obtained using the above-mentioned sialated Lewis X as the immunogen; hereinafter referred to as CSLEX1)-RPHA reagent was used. When alpha fetoprotein (hereinafter referred to as AFP)-RPHA reagent was used, AFP was not detected.

Table 1 Information about the Surgical SpecimenPatient : 64 years old, male

Blood group : A-group, RhD(+)

Tumor : 7 $\times$ 6 $\times$ 7 cm, Stage III

Pathological findings : Cholangiocellular carcinoma

Cholangioma/bile duct cancer

Tumor markers AFP : Negative

Tumor markers CEA : 10.2 ng/ml

Tumor markers $\beta_2$ Microglobulin : 1.8 mg/ml

Tumor markers Ferritin : 167.5 ng/ml

Tumor markers CA19 9 : 6200 U/ml

(1-ii) Immunization

HEK cells (1.5 to 2.0 $\times$ $10^7$ cells) which had grown over the almost entire area of the 150 cm² cultivation surface of a flask were incubated together with anti-human leukocyte mouse antibodies at room temperature for 1 hour; thereafter, they were intraperitoneally administered to a BALB/C line mouse for immunization. Two weeks after 4 times of immunization at intervals of 10 days to 1 month, a blood sample was taken and blood antibody titer was measured. Three months later, 2.0 $\times$ $10^7$ HEK cells were intraperitoneally administered for further immunization. Four days after the final immunization, the spleen was resected from the mouse to be used for the cell fusion described below.

(2) Cell fusion and cloning:

9.0 $\times$ $10^7$ of the above-mentioned mouse splenic cells and 3.9 $\times$ $10^7$ cells of mouse myeloma P3U1 [Current Topics in Microbiology and Immunology, 81, 1 (1978)] were mixed together and reaction was carried out for 1 minute using 45% polyethylene glycol (average molecular weight: 4,000) by a partial modification of the method of Köhler et al. [Immunological Method, Academic Press, New York, 391 (1979) to thereby achieve cell fusion.

The treated cells were inoculated to a 96-well microplate and cultured in HAT medium (Table 2) for 9 to 14 days; thereafter, they were transferred to HT medium (Table 2). When their growth became sufficient to enable cultivation in a flask (25 cm²), they were cultured in D-MEM medium (Table 2). The antibody titer of the culture supernatant of each well in which proliferation occurred was determined by the ELISA, and cloning of the desired hybridomas was conducted from an appropriate well by limiting dilution analysis.

A microtiter plate was inoculated with 25,000/well mouse peritoneal exudate cells; then, the hybridomas were diluted with D-MEM medium to 10, 5, 2.5 and 1 cell/0.1 ml, and 0.1 ml of each of these dilutions was inoculated to the microtiter plate and cultured. Four days later, 0.1 ml of D-MEM medium was added; thereafter, the half amount of the medium was exchanged at intervals of 4 to 7 days. Within 10 to 20 days after the initiation of cultivation, visually recognizable colonies were formed and clones were obtained.

Table 2

| (a) D-MEM medium: |  |
| --- | --- |
| Dulbecco's modified Eagle medium (hereinafter abbreviated D-MEM), produced by Nissui Seiyaku Co. Prepared by supplementing D-MEM with the following additives: |  |
| (Final concentration) | (Constituent) |
| 10% | Equine serum (Flow Co.) |
| 300 mg/ℓ | L-glutamine |
| 110 mg/ℓ | Sodium pyruvate |
| 100 I.U./ml | Penicillin |
| 100 μg/ml | Streptomycin |
| 3.5 g/ℓ | Glucose |
| 3.7 g/ℓ | $NaHCO_3$ |
| (b) HAT medium: |  |
| Prepared by supplementing D-MEM with the following additives: |  |
| $1 \times 10^{-4}$ M | Hypoxanthine |
| $4 \times 10^{-7}$ M | Aminopterin |
| $1.6 \times 10^{-5}$ M | Thymidine |
| (c) HT medium: |  |
| Medium of the same composition as of HAT medium except that aminopterin is not contained. |  |

(3) Method of screening

The obtained hybridomas were subjected to screening of clones which produce the desired monoclonal antibody as follows:

(a) Description of the method

The ELISA was performed as follows:
The hybridoma culture supernatant was added to a microplate coated with an antigen (one of various established cancer cell lines, partially purified cancer-associated antigens or normal cells), and reaction was carried out at 37°C for 1 hour. After washing the microplate, peroxidase-labeled anti-mouse immunoglobulin (IgG + IgA + IgM) rabbit antibodies were added, and reaction was carried out at 37°C for 1 more hour. After washing to remove excess labeled antibodies, an o-phenylenediamine solution was added, and reaction was carried out at room temperature for 30 minutes. Then, the reaction was stopped by adding 2M sulfuric acid and absorbance at 490 nm was determined. Reactivities with various cells were examined by this method. Cross reactivities with CEA were determined by the PHA method using CEA-sensitized blood cells.

Antibodies to KM01 antigen and CSLEX1 antigen were measured by the HAI method using cancer cell culture supernatants confirmed to respectively contain KM01 antigen and CSLEX1 antigen and the reagents KM01-RPHA and CSLEX1-RPHA. That is, after reaction at 37°C for 1 hour between the hybridoma culture supernatant and properly diluted cancer cell (HEK) culture supernatant, the amounts of remaining antigens were measured by means of respective RPHA reagents (Table 4).

To know which of the secretory antigen and cell surface antigen of HEK the monoclonal antibody recognizes, it was examined whether the reaction between the monoclonal antibody and HEK cells themselves are inhibited by HEK culture supernatant. The specific procedure of the inhibition test using the ELISA is as follows: The hybridoma culture supernatant is titrated by the ELISA and an appropriate dilution rate is determined on the basis of the results of the titration. Then, the equal amount of each dilution prepared at a given dilution rate ($1:5$, $1:5^2$ . . . $1:5^n$) from the original solution which is a 5 to 25-fold concentrate of the HEK culture supernatant was added to each appropriate dilution of the hybridoma culture supernatant. This was followed by 1 hour of incubation at 37°C. Thereafter, the remaining antibody activity was assayed by the ELISA using HEK as a target.

(b) Flow of the screening

Primary screening: By the ELISA using the target cell (HEK) and fetal lung-derived fibroblast (Flow-2000), wells which are positive for HEK and negative for Flow-2000 were selected.

Secondary screening: The hybridomas selected in the primary screening were cloned 2 to 3 times. The culture supernatants thereof were examined for reactivity with various cancer-derived established cell lines and normal tissue-derived cell lines, and were also subjected to the inhibition test using the enzyme antibody technique to thereby identify which of secretory antigen and cell membrane surface antigen the cloned lines reconize.

Thus, 62 wells were selected in the primary screening from the 960 wells ($9 \times 10^4$ splenic cells per well) at the time of cell fusion.

As a result of the secondary screening, it was found that an antibody titer against HEK was held in 53 wells, KM01-like antibody was produced in 10 wells, anti-CEA antibody seemed to be produced in 11 wells and CSLEX1-like antibody seemed to be produced in none of the wells.

(4) Recovery and purification of the monoclonal antibodies

Three clones obtained in the above-mentioned screening were each intraperitoneally transplanted in an amount of 2.0 to 3.0 $\times$ $10^5$ cells/mouse to at least 4-week-old BALB/C mice (male) previously primed with 0.5 ml/animal pristane; 10 to 14 days later, ascites fluid containing a high concentration of the monoclonal antibody was collected.

After diluting this ascites fluid to a 5 to 10-fold volume with a 0.9% NaCl solution, ammonium sulfate was added to 40% saturated concentration and the precipitate fraction was separated. This precipitate fraction was dissolved in an as small as possible amount of a 0.9% NaCl solution, and dialyzed against a 0.9% NaCl solution as the external liquid. After the completion of dialysis, high performance liquid chromatography (TSK-Gell G-3000SW) was conducted to give an immunoglobulin fraction, which was used as the purified monoclonal antibody.

(5) Characteristics of the monoclonal antibodies

The characteristics of the monoclonal antibodies produced by the clones thus screened are as shown in Tables 3 and 5. Immunoglobulin classes were determined by the Ouchterlony method.

The cell lines subjected to the screening were obtained as follows:

Bile duct cancer-derived cell line HEK was obtained as described above. The cell lines PA-1, HEp-2, KB, PANC-1, MIAPaCa-2, COLO-201, COLO-320DM and ZR75-1 were obtained from Dainippon Pharmaceutical Co. Ltd.; the cell lines NRC-12, NBT-2, PC-1, PC-3, PC-9, PC-10, MKN-28, MKN-45 and KATO-III were obtained from Immunological and Biological Laboratory; TE-1, TE-2, TE-4, TE-5, TE-6, TE-7, TE-8, TE-9, TE-10 and TE-11 were obtained from Tohoku University; the line ISHIKAWA was obtained from Osaka University; G-415 was obtained from Tsukuba University; and PA was obtained from Kinki University.

All these cancer cell lines were subcultured in RPMI-1640 medium containing 10% fetal bovine serum (Filtron), 2 mM L-glutamine, 1 mM sodium pyruvate, 100 IU/ml penicillin and 100 μg/ml streptomycin.

The cell lines Flow-2000, Flow-11000, CCD$_{18}$-Co, CCD$_{45}$-SK, WISH and FL were obtained from Dainippon Pharmaceutical Co. Ltd. HKG and NK-3 were obtained from the Green Cross Corporation.

All these cell lines were subcultured in MEM medium containing 10% fetal bovine serum (Filtron), 100 IU/ml penicillin and 100 g/ml streptomycin.

Mouse myeloma P3U1 line was obtained from Dainippon Pharmaceutical Co. Ltd., and it was subcultured in D-MEM medium containing 10% equine serum (Flow Co.), 2 mM L-glutamine, 1 mM sodium pyruvate, 100 IU/ml penicillin and 100 μg/ml streptomycin.

The ELISA used in this invention is that cells are directly utilized as the antigen in accordance with the method of Kennett et al. [Monoclonal Antibody, Plenum Press, New York and London, 376, (1980)].

New York and London, <u>376</u>, (1980)].

Table 3  Properties of Antibodies

| Antibody | Immunoglobulin type | Titer as determined by enzyme antibody technique (cell supernatant) | Recognition antigen |
|---|---|---|---|
| AF6<br>FD5<br>HH9 | IgM($\kappa$) | 1 : 3125-15625<br>1 : 3125-15625<br>1 : 3125-15625 | Secretory antigen |

Table 4  Reactivities with Tumor Markers

| Antibody \ Item | KM01 | AF6 | FD5 | HH9 |
|---|---|---|---|---|
| KM01 | + | + | + | + |
| CEA | − | − | − | − |
| CSLEX1 | − | − | − | − |

Note: + indicates being positive and − indicates being negative.

Table 5  Reactivities with Various Cancer Cell Lines

| Cancer cell line | Derivation | KM01 | AF6 | FD5 | HH9 |
|---|---|---|---|---|---|
| TE-1 | Esophageal cancer, highly differentiated | + | + | + | + |
| TE-2 | Esophageal cancer, poorly differentiated | +++ | +++ | +++ | +++ |
| TE-3 | Esophageal cancer, highly differentiated | + | + | − | + |
| TE-4 | Esophageal cancer, highly differentiated | − | ± | − | − |
| TE-5 | Esophageal cancer, poorly differentiated | + | ± | + | + |
| TE-6 | Esophageal cancer, moderately differentiated | − | + | − | − |
| TE-7 | Esophageal cancer | +++ | +++ | +++ | +++ |
| TE-8 | Esophageal cancer, highly differentiated | − | − | − | − |

| | | | | | |
|---|---|---|---|---|---|
| TE-9 | Esophageal cancer, poorly differentiated | +++ | +++ | +++ | +++ |
| TE-10 | Esophageal cancer, highly differentiated | + | + | +++ | + |
| TE-11 | Esophageal cancer | − | ± | − | − |
| PC-1 | Lung cancer, poorly differentiated | − | ± | − | − |
| PC-3 | Lung cancer, moderately differentiated | +++ | +++ | +++ | +++ |
| PC-9 | Lung cancer, differentiated | − | − | − | + |
| PC-10 | Lung cancer, moderately differentiated | − | − | − | − |
| MKN-28 | Gastric cancer, highly differentiated | ++ | + | ± | ++ |
| MKN-45 | Gastric cancer, poorly differentiated | + | + | +++ | ++ |
| KATO-III | Gastric cancer, signet ring cells | +++ | +++ | +++ | +++ |
| COLO-201 | Colorectal cancer | +++ | +++ | +++ | +++ |
| COLO-320DM | Colorectal cancer | − | + | − | − |
| G-415 | Gallbladder cancer poorly differentiated | − | − | − | ± |
| ZR75-1 | Breast cancer | − | ± | − | − |
| PA | Malignant melanoma | − | ± | − | − |
| NRC-12 | Renal cellular cancer | − | − | − | + |
| PA-1 | Ovarian teratoma | ± | ± | | ++ |
| HEp-2 | Laryngeal cancer | − | − | − | − |
| KB | Rhinopharyngeal cancer | − | − | − | − |
| NBT-2 | Bladder cancer, transitional epithelium | − | − | − | ± |
| HEK | Bile duct cancer | +++ | ++ | +++ | +++ |
| PANC-1 | Pancreatic cancer | − | − | − | − |
| MIAPaCa-2 | Pancreatic cancer | − | − | − | − |
| ISHIKAWA | Endometrial cancer | + | ++ | + | + |
| $CCD_{45}$-SK | Normal skin | − | + | − | − |
| $CCD_{18}$-Co | Colon | − | + | − | − |
| Flow-2000 | Fetal lung | − | ± | − | − |
| Flow-11000 | Fetal small intestine | − | + | − | − |
| NK-3 | Kidney, primary culture | − | ± | − | − |
| HKG | Kidney | ± | ± | + | +++ |
| WISH | Amnion | − | − | − | − |
| FL | Amnion | − | − | − | − |

Note: In respect to CELISA reactivity, +++, ++ and + indicate being positive; ± indicates being pseudopositive; − indicates being negative. The $OD_{490}$ values in the CELISA method are as follows:

- : Less than 0.05
± : 0.05 or more and less than 0.1
+ : 0.1 or more and less than 0.4
++ : 0.4 or more and less than 0.7
+++ : 0.7 or more

## Claims

1. A monoclonal antibody having the following characteristics which are produced by hybridomas prepared using a bile duct cancer-derived established cancer cell line (HEK) as the immunogen.

    1) Ig class (subclass): IgM(x)

    2) Type of recognizing antigen: Secretory antigen

    3) Reactivity with cancer cells: Positive for at least esophageal cancer cell line TE-1, esophageal cancer cell line TE-2, esophageal cancer cell line TE-7, esophageal cancer cell line TE-9, esophageal cancer cell line TE-10, lung cell line PC-3, gastric cancer cell line MKN-45, gastric cancer cell line KATO-III, bile duct cancer cell line HEK, endometrial cancer cell line ISHIKAWA and colorectal cancer cell line COLO-201.

2. The monoclonal antibody as claimed in claim 1 whose reactivity with cancer cells is also positive for esophageal cancer cell line TE-3, esophageal cancer cell line TE-6, gastric cancer cell line MKN-28 and colorectal cancer cell line COLO-320DM.

3. The monoclonal antibody as claimed in claim 1 whose reactivity with cancer cells is also positive for esophageal cancer cell line TE-5.

4. The monoclonal antibody as claimed in claim 1 whose reactivity with cancer cells is also positive for esophageal cancer cell line TE-3, lung cancer cell line PC-9, renal cancer cell line NRC-12 and ovarian teratoma cell line PA-1.

5. The monoclonal antibody as claimed in claim 1 in which said antibody-producing cell is a splenic cell, a lymph node cell or a B-lymphocyte derived from an animal immunized with the antigen obtained from HEK.

6. The monoclonal antibody as claimed in claim 1 in which said immunogen is HEK established from a bile duct cancer-derived cell.

7. The monoclonal antibody as claimed in claim 5 in which said immunized animal is a mouse, rat, horse, goat or rabbit.